# EUROPÄISCHE PATENTSCHRIFT

(11) **EP 1 637 065 B1**
(45) Veröffentlichungstag und Bekanntmachung des Hinweises auf die Patenterteilung: **11.04.2012**
(21) Anmeldenummer: 05016840.0
(22) Anmeldetag: 03.08.2005
(51) Int. Cl.: A61B 1/12, A61B 1/00

(54) **Endoskopisches Instrument**
Endoscopic instrument
Instrument endoscopique

(30) Priorität: 21.09.2004 DE 202004014828 U
(43) Veröffentlichungstag der Anmeldung: 22.03.2006
(73) Patentinhaber: Richard Wolf GmbH, 75438 Knittlingen (DE)
(72) Erfinder: Hipp, Klaus-Peter, 75038 Grossvillars (DE); Bonnet, Ludwig, 7134 Knittligen (DE); Fritz, Michael, 76199 Karlsruhe (DE); Ernst, Thomas, 75057 Kürnbach (DE); Riedmiller, Hubertus, Prof. Dr., 97082 Würzburg (DE)
(74) Vertreter: Hemmer, Arnd

(56) Entgegenhaltungen:
- DE-A1- 10 138 331
- DE-A1- 19 826 311
- DE-U1- 20 004 329
- DE-U1-202004 014 828
- US-A- 5 287 845
- US-A- 5 486 155
- US-B1- 6 358 200

## Beschreibung

Die Erfindung betrifft ein endoskopisches Instrument mit den im Oberbegriff des Anspruchs 1 angegebenen Merkmalen.

Endoskopische Instrumente, wie sie beispielsweise zur Resektoskopie eingesetzt werden, weisen im Allgemeinen einen Schaft auf, durch den Arbeitsgeräte typischerweise in Form eines Arbeitseinsatzes geführt werden, die proximalseitig an dem Schaft angebunden werden. Über entsprechende Anschlüsse wird bei diesen Instrumenten eine Spülflüssigkeit in den Schaft eingeleitet, dem Operationsgebiet zugeführt und anschließend zusammen mit Geweberesten wieder abgesaugt.

Generell unterscheidet man bei derartigen endoskopischen Instrumenten solche mit einer fortwährenden Spülung von Instrumenten mit intermittierender Spülung.

Die Schäfte der Instrumente mit einer fortwährenden Spülung sind doppelwandig ausgebildet und werden als sogenannte "Continuos-Flow"-Schäfte bezeichnet. Bei diesen Schäften wird dem Operationsgebiet fortwährend über ein inneres Schaftrohr Spülflüssigkeit zugeführt, während Spülflüssigkeit gleichzeitig über einen von dem inneren und einem äußeren Schaftrohr gebildeten Ringspalt wieder abgesaugt wird. Es ist bekannt, endoskopische Instrumente mit einem "Continuos-Flow"-Schaft so auszubilden, dass ein in dem inneren Schaftrohr geführter Arbeitseinsatz zusammen mit dem inneren Schaftrohr drehbar ist, während das äußere Schaftrohr nicht verdreht wird. Dies ist vorteilhaft, da ein Verdrehen des Außenschaftes das ihn umgebende Gewebe traumatisieren könnte. Nachteilig steht dem aber gegenüber, dass der Außendurchmesser der "Continuous-Flow"-Schäfte konstruktionsbedingt sehr groß ist und das den Schaft umgebende Körpergewebe auf andere Weise belastet.

Solche endoskopische Instrumente mit einem "Continuous-Flow"-Schaft beschreiben US 5,287,845 A und US 5,486,155 A. Bei diesen Instrumenten ist jeweils ein Innenschaft drehbar mit einem Außenschaft verbunden. An dem Außenschaft ist ein Zufuhranschluss zum Zuführen einer Spülflüssigkeit angeordnet, so dass ein Zufluss vom Zuflussanschluss über einen Ringkanal zwischen Außen- und Innenschaft durch Öffnungen des Innenschaftes in dessen Innenraum und zu dessen distalem Ende erfolgt. Die Verbindung von Außen- und Innenschaft in ihrer axialen Position zueinander erfolgt bei diesen Instrumenten über eine Verriegelung. In US 5,287,845 A dient die Verriegelung gleichzeitig als Drehlager, während in der in US 5, 486,155 A beschriebenen Verriegelung ein Arbeitseinsatz drehbar gelagert ist.

Den Nachteil eines sehr großen Außendurchmessers weisen endoskopische Instrumente mit intermittierender Spülung, bei denen eine gleich große Schlinge zum Abtragen von Gewebe wie bei den "Continuous-Flow"-Schäften eingesetzt wird, nicht auf. Solche Instrumente haben einen einfachen Hohlschaft, so dass der Außendurchmesser deutlich kleiner als bei "Continuous-Flow"-Schäften ausgebildet sein kann. Nachteilig ist bei diesen Instrumenten wiederum, dass der Arbeitseinsatz nur zusammen mit dem Schaft verdreht werden kann.

Diesen Nachteil suchen bekannte endoskopische Instrumente mit intermittierender Spülung zu vermeiden, bei denen der Arbeitseinsatz in Verbindung mit dem Hohlschaft drehbar ist und die Leitungsanschlüsse mit den daran angebundenen Schläuchen bei einer Drehung des Arbeitseinsatzes stehen bleiben. Eine Traumatisierung des Gewebes, beispielsweise einer Harnröhre, wird damit jedoch nicht reduziert wie gewünscht.

DE 200 04 329 beschreibt ein Instrument, mit einer an dem proximalen Ende eines Hohlschaftes befestigbaren Arbeitskappe, in der ein Arbeitseinsatz mit einem Spülanschluss drehbar gelagert ist.

Vor diesem Hintergrund liegt der vorliegenden Erfindung die Aufgabe zugrunde, ein endoskopisches Instrument zu schaffen, welches die oben beschriebenen Nachteile vermeidet und einfach zu reinigen ist.

Diese Aufgabe wird durch ein endoskopisches Instrument mit den in Anspruch 1 angegebenen Merkmalen gelöst. Vorteilhafte Weiterbildungen der Erfindung ergeben sich aus den Unteransprüchen der nachfolgenden Beschreibung und den Zeichnungen.

Das erfindungsgemäße endoskopische Instrument ist typischerweise intermittierend spülend ausgebildet. Es weist einen Hohlschaft, der proximalseitig mit einem Anschlussteil mit zumindest einem Saug- und/oder Spülanschluss versehen ist sowie ein Kupplungsteil zum Festlegen eines Arbeitseinsatzes auf, wobei das Kupplungsteil gegenüber dem Anschlussteil drehbar gelagert ist. Des Weiteren weist das Instrument eine ringförmige Axialdichtung auf, welche zwischen dem Anschlussteil und dem Kupplungsteil angeordnet ist. Das Kupplungsteil weist Mittel zur lösbaren Verbindung an dem Anschlussteil des Instrumentes auf, wobei das Kupplungsteil drehbar gegenüber den Verbindungsmitteln zum Anschlussteil ausgebildet ist. Distalseitig weist das Kupplungsteil eine Axialdichtung auf, die mit dem Kupplungsteil mitdreht und zur dichtenden Anlage an einer Stirnfläche des Anschlussteils ausgebildet ist.

Proximalseitig ist an dem Anschlussteil ein Kupplungsteil angeordnet. Das Kupplungsteil dient zum Festlegen des Arbeitseinsatzes, der durch das Schaftrohr geführt wird. Gemäß der Erfindung ist das Kupplungsteil so lösbar mit dem Anschlussteil verbunden, dass das Kupplungsteil gegenüber dem Anschlussteil drehbar ist. Dies ermöglicht ein Verdrehen des Arbeitseinsatzes während eines endoskopischen Eingriffs, ohne dass der Hohlschaft des endoskopischen Instruments mitgedreht wird. Auf diese Weise kann eine Belastung des Patienten, beispielsweise eine Traumatisierung des den Schaft umgebenden Körpergewebes, zu der es bei Drehung des Schaftes kommen kann, verhindert werden. Das

Anschlussteil wird bei dem erfindungsgemäßen endoskopischen Instrument bei Drehung des Arbeitseinsatzes nicht mitdreht, so dass ein Operateur z.B. nicht durch einen sich verdrehbaren Schlauch bzw. Schläuche, die an dem Anschluss an dem Anschlussteil angebunden sind, behindert wird. Die lösbare Verbindung von Kupplungsteil und Anschlussteil ermöglicht er ferner, dass das Kupplungsteil beispielsweise zu Reinigungszwecken schnell und einfach von dem Anschlussteil getrennt und später wieder zusammengefügt werden kann.

Über eine ringförmige Axialdichtung wird das Kupplungsteil gegenüber dem Anschlussteil abgedichtet. Die Axialdichtung ist so angeordnet, dass sie ab dem Kupplungsteil und an dem Anschlussteil dichtend anliegt. Neben der Axialdichtung werden bei dem erfindungsgemäßen endoskopischen Instrument keine weiteren Dichtungen benötigt, um das Anschlussteil gegenüber dem Kupplungsteil abzudichten. Die Axialdichtung liegt an einer proximalseitigen Stirnfläche des Anschlussteils und einer distalseitigen Stirnfläche des Kupplungsteils an.

Die Axialdichtung ist distalseitig an dem Kupplungsteil angeordnet, und dreht mit dem Kupplungsteil mit, während sie an einer Stirnfläche des Anschlussteils dichtend anliegt. Wegen der Drehung der Axialdichtung relativ zu dem feststehenden Anschlussteil ist die Axialdichtung vorteilhaft aus einem Material auszubilden, welches neben sehr guten Dichteigenschaften auch einen niedrigen Reibungskoeffizienten aufweist. Vorzugsweise ist die Axialdichtung aus einem elastomeren Kunststoff ausgebildet, es sind aber auch anderen Materialien denkbar, die die oben erwähnten Werkstoffeigenschaften aufweisen.

Distalseitig weist das Kupplungsteil bevorzugt einen hohlzylindrischen Abschnitt auf, der abgestuft ausgebildet ist. An einer Stirnfläche des abgestuften Teils des hohlzylindrischen Abschnitts wird die Axialdichtung vorteilhafterweise abgestützt. Die Stirnfläche ist ringförmig und wird durch eine an dem distalen Ende des hohlzylindrischen Abschnitts angeordnete Stufung der Außenkontur des Kupplungsteils gebildet. Auf dieser Stirnfläche liegt die Axialdichtung so auf, dass sie sowohl in radialer als auch in axialer Richtung an dem Kupplungsteil festgelegt ist. Die Stirnfläche ist vorteilhaft so weit von dem distalen Ende des Kupplungsteils beabstandet, dass eine an dieser Stirnfläche anliegende Axialdichtung die Stufe und damit das Kupplungsteil in axialer Richtung überragt. Auf diese Weise kann die Axialdichtung, wenn das Kupplungsteil an dem Anschlussteil angebunden ist, zwischen der Stirnfläche des Hohlzylindrischen Abschnitts und einer Stirnfläche am Anschlussteil dichtend eingespannt werden und das Kupplungsteil gegenüber dem Anschlussteil abgedichtet werden. Des Weiteren hat diese Anordnung den Vorteil, dass die Axialdichtung in einfacher Weise nach Lösen des Kupplungsteils ausgetauscht oder zu Reinigungszwecken entfernt und eingebaut werden kann.

In einer vorteilhaften Ausführungsform des erfindungsgemäßen endoskopischen Instruments weist das Anschlussteil ein Schieberventil auf. Dieses verbindet den Hohlschaft wahlweise mit einem Spül- oder einem Saugkanal. In diesem Fall sind an dem Anschlussteil zwei Anschlüsse angeordnet. Dabei wird die proximale Stirnseite des Drehschiebers des Schieberventils gleichzeitig als Anlage- und Dichtfläche für die Axialdichtung genutzt. Der Drehschieber des Schieberventils ist flüssigkeitsdicht so in dem Anschlussteil angeordnet, dass er jeweils nur einen dieser Anschlüsse freigibt, d.h., dass er entweder nur den Spülkanal oder den Saugkanal, die jeweils an einem dieser Anschlüsse angebunden sind mit dem Hohlschaft verbindet. Der Operateur kann durch Betätigen des Schieberventils den Hohlschaft mit dem Spülanschluss verbinden oder mit dem Sauganschluss verbinden. Des Weiteren ist es möglich den Drehschieber in eine neutrale Stellung zu stellen, in der beide Anschlüsse von dem Drehschieber verschlossen werden. Vorzugsweise ist der Drehschieber des Schieberventils so an dem proximalen Ende des Anschlussteils angeordnet, dass er zum Reinigen leicht aus dem Anschlussteil ausgebaut werden kann. Eine proximale Stirnfläche des Drehschiebers kann dann die Anlagefläche für die an dem Kupplungsteil angeordnete Axialfläche bilden, auf der sich die Axialdichtung distalseitig abstützt.

Das Kupplungsteil ist gegenüber den Mitteln zur lösbaren Verbindung des Kupplungsteils an dem Anschlussteil drehbar gelagert.

Die Verbindungsmittel selbst sind dann drehfest an dem Anschlussteil angebunden.

Bevorzugt weisen die Mittel zur lösbaren Verbindung von Kupplungsteil und Anschlussteil eine am Kupplungsteil angeordnete Überwurfmutter und ein am Anschlussteil angeordnetes Außengewinde auf. Die Überwurfmutter und das am Anschlussteil angeordnete Außengewinde sind dann zweckmäßigerweise gegen einen Anschlag derart verschraubbar, dass das Kupplungsteil gegenüber dem Anschlussteil stets frei drehbar ist.

Der Anschlag kann sowohl an der Überwurfmutter als auch an dem Anschlussteil angeordnet sein, ist aber vorzugsweise an dem Anschlussteil ausgebildet. Wird die Überwurfmutter an dem Anschlussteil aufgeschraubt, begrenzt der Anschlag den gemeinsamen Gewindegang von Überwurfmutter und Außengewinde derart.

Die Überwurfmutter ist zwischen einer Stirnfläche am Außenumfang des Kupplungsteils und einem an einer Umfangsfläche des hohlzylindrischen Ansatzes des Kupplungsteils angeordneten Fixierring formschlüssig gehalten. Die Überwurfmutter, die den Außenumfang des Kupplungsteils im Bereich des hohlzylindrischen Ansatzes mit geringem Spiel übergreift, ist derart an dem Kupplungsteil angebunden, dass das Kupplungsteil gegenüber der Überwurfmutter drehbar, jedoch formschlüssig auf diesem gehalten ist. Da die Überwurfmutter ohnehin drehbar sein muss, ist es besonders günstig hier auch die drehbare Lagerung zwischen Kupplungsteil und Anschlussteil vorzusehen.

Die Stirnfläche am Außenumfang des Kupplungsteils kann vorteilhaft durch einen Absatz an der Außenseite des Kupplungsteils ausgebildet sein, der der Überwurfmutter an dem Kupplungsteil eine proximalseitige Auflagefläche bietet und somit das Axiallager zur Aufnahme von Kräften in proximaler Richtung bilden. An der distalseitigen Stirnfläche der Überwurfmutter ist der Fixierring mit geringem axialem Spiel auf dem Kupplungsteil an der Umfangsfläche des hohlzylindrischen Ansatzes drehbar gelagert.

Zur Befestigung des Fixierrings an der Umfangsfläche des hohlzylindrischen Ansatzes weist der Fixierring vorteilhaft ein Innengewinde auf, mit dem er in ein Außengewinde, welches an einer Umfangsfläche des hohlzylindrischen Ansatzes des Kupplungsteils vorgesehen ist, eingreift. Dies ermöglicht ein leichtes Anbringen der Überwurfmutter an dem Kupplungsteil. Der Fixierring bildet dabei einen Anschlag, der das Lagerspiel festlegt, mit dem die Überwurfmutter am Kupplungsteil drehbar gelagert ist.

In einer vorteilhaften Ausführungsform der Erfindung fluchten der Hohlschaft des endoskopischen Instruments und die dem Hohlschaft zugewandte distalseitige Öffnung des Kupplungsteils, wobei die proximalseitige Öffnung des Kupplungsteils, exzentrisch zu der distalseitigen Öffnung angeordnet ist. Diese Ausführungsform ist vorzugsweise für den Einsatz solcher Arbeitseinsätze vorgesehen, welche bezogen auf eine gemeinsame Längsachse nicht über die gesamte Länge rotationssymmetrisch sind. Diese Arbeistseinsätze weisen beispielsweise einen Abschnitt auf, an dem sie an dem Kupplungsteil festgelegt sind, der exzentrisch zu dem Abschnitt des Arbeitsabschnitts ausgerichtet ist, der in dem Hohlschaft geführt wird. Des Weiteren weisen solche Arbeitseinsätze in diesen Abschnitten unterschiedliche Durchmesser auf. Korrespondierend zu der Form dieser Arbeitseinsätze ist das Kupplungsteil gestaltet, so dass bei dem endoskopischen Instrument nur die für dieses Instrument vorgesehenen Arbeitseinsätze eingesetzt werden können. Um trotzt der Exzentrizität der Arbeitseinsätze diese mit dem Kupplungsteil gegenüber dem Anschlussteil bzw. gegenüber dem Hohlschaft verdrehen zu können, ist es vorgesehen, dass der Hohlschaft und die dem Hohlschaft zugewandte distalseitige Öffnung des Kupplungsteils fluchten, d.h., eine gemeinsame Mittelachse aufweisen.

Es sei an dieser Stelle darauf hingewiesen, dass das erfindungsgemäße Kupplungsteil nicht nur bei endoskopischen Instrumenten mit einem einfachen Hohlschaft, sondern auch bei solchen Instrumenten eingesetzt werden kann, die fortwährend gespült werden und demgemäß einen doppelwandigen "Continuos-Flow"-Schaft aufweisen, so dass diese Instrumente dann auch von dem geringen Reinigungs- und Wartungsaufwand, den das Kupplungsteil erfordert, profitieren.

Nachfolgend ist die Erfindung anhand eines in der Zeichnung dargestellten Ausführungsbeispiels erläutert. Es zeigen
- Fig. 1.: einen Längsschnitt eines erfindungsgemäßen endoskopischen Instruments,
- Fig. 2.: in vergrößerter Darstellung das Anschlussteil mit daran angeordnetem Kupplungsteil des Instruments nach Fig. 1 und
- Fig. 3.: das Anschlussteil gemäß Fig.2 in Seitenansicht mit getrennt angeordnetem Kupplungsteil im Schnitt.

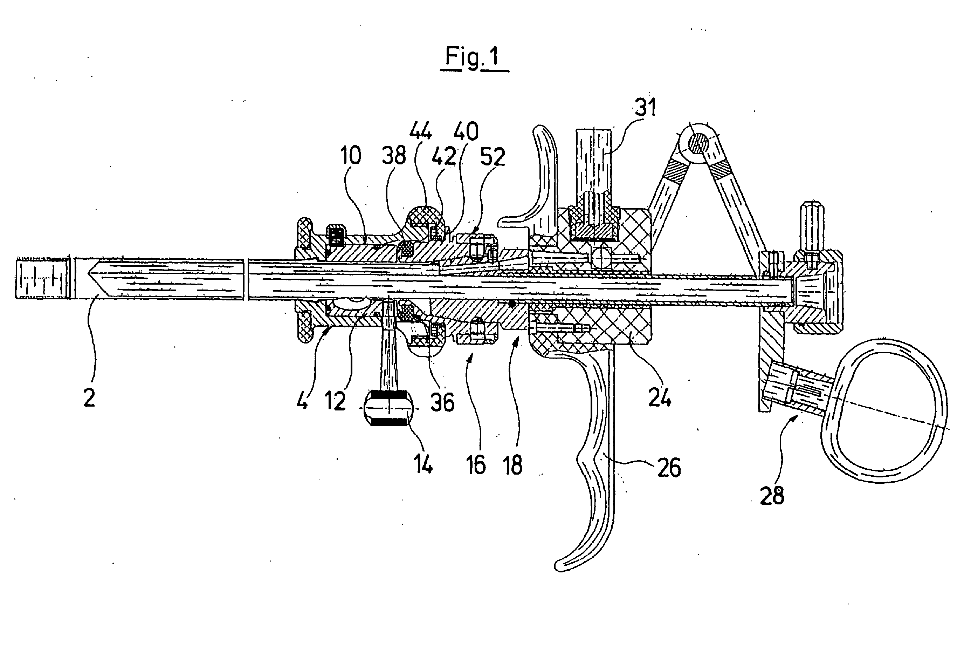

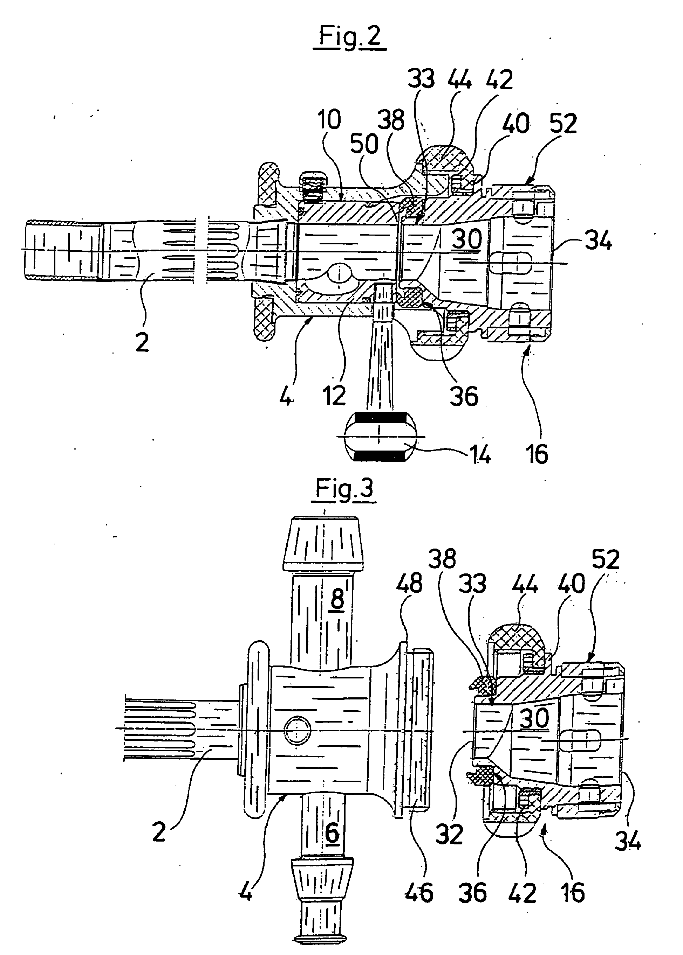

Bei dem in Fig. 1 dargestellten endoskopischen Instrument handelt es sich um ein Resektoskop mit einem distalseitigen Hohlschaft 2, an dessen proximalen Ende ein Anschlussteil 4 angeordnet ist. Wie aus Fig. 3 ersichtlich, weist das Anschlussteil 4 sowohl einen Leitungsanschluss 6 zum Einleiten einer Spülflüssigkeit in den Hohlschaft 2 als auch einen Leitungsanschluss 8 zum Absaugen der Spülflüssigkeit aus dem Hohlschaft 2 auf. Mittels eines in dem Anschlussteil 4 angeordneten Schieberventils 10 kann wahlweise der Leitungsanschluss 6 oder der Leitungsanschluss 8 verschlossen oder freigegeben werden. In einer Mittelstellung verschließt das Schieberventil 10 sowohl den Leitungsanschluss 6 als auch den Leitungsanschluss 8. Hierzu weist das Schieberventil 10 einen Drehschieber 12 auf, der mit Hilfe eines Hebels 14 so um die Längsachse des Hohlschafts 2 drehbar ist, dass in einer ersten Stellung des Drehschiebers 12 der Anschluss 6 freigegeben und der Anschluss 8 verschlossen wird und in einer zweiten Stellung der Anschluss 6 verschlossen und der Anschluss 8 freigegeben wird.

Proximalseitig schließt sich an das Anschlussteil 4 ein Kupplungsteil 16 an. An diesem Kupplungsteil 16 ist ein an sich bekannter und daher hier nicht im einzelnen beschriebener Arbeitseinsatz 18 festgelegt, in dem eine nicht dargestellte Optik sowie eine HF Schneidschlinge geführt werden. Das proximale Ende der Schneidschlinge ist in einem Schlosskörper 24 festgelegt, welcher auch den elektrischen Anschlusskontakt 31 trägt. Mittels an dem Schlosskörper 24 angeordneten Handhaben 26 und 28 kann die Schneidschlinge in Längsrichtung verfahren werden.

Das Kupplungsteil 16 weist eine zu der Kontur des Arbeitseinsatzes 18 komplementäre Durchbrechung 30 auf. Diese Durchbrechung 30 hat eine distalseitge Öffnung 32 und eine proximalseitige Öffnung 34, wobei die Öffnung 34 exzentrisch zur Öffnung 32 angeordnet ist, also nur die Öffnung 32 zum Schaft 2 fluchtet.

Im Bereich der dem Anschlussteil 4 zugewandten Öffnung 32 ist stirnseitig des im Wesentlichen zylindrischen Kupplungsteils 16 ein hohlzylindrischer Ansatz 33 gebildet, an dem ein Absatz 36 vorgesehen. Auf diesem Absatz 36 ist ein Dichtring 38 so angeordnet, dass er an einer von dem Absatz 38 gebildeten Stirnfläche und an der von dem Absatz gebildeten Umfangsfläche dicht anliegt. Der Dichtring 38 wird so von dem Absatz 36 sowohl in radialer als auch in axialer Richtung abgestützt und ist auf diese Weise drehfest an dem Kupplungsteil 16 angebunden.

In proximaler Richtung hinter dem Absatz 36 ist am Außenumfang des Kupplungsteils 16 ein zweiter Absatz 40 gebildet. Zwischen diesem Absatz 40 und einer Zweilochmutter 42, die einen Fixierring bildet, ist eine Überwurfmutter 44 drehbar gelagert. Die Zweilochmutter 42 weist ein Innengewinde auf, mit dem sie auf einem Außengewinde verschraubt, welches an einem distalseitig des Absatzes 40 anschließenden Umfangsbereich des Kupplungsteils 16 angeordnet ist. Sie weist stirnseitig einen Anschlagring auf, der so ausgebildet ist, das die Überwurfmutter 44, wenn die Zweilochmutter 42 gegen den Absatz 40 verschraubt ist, sowohl radial als auch axial mit geringem Spiel gelagert ist. Die Überwurfmutter 44 ist so gegenüber dem Kupplungsteil 16 drehbar und bildet sowohl ein Axial- als auch ein Radiallager für das Kupplungsteil 16.

Mittels der Überwurfmutter 44 ist das Kupplungsteil 16 mit dem Anschlussteil 4 verbindbar. Hierzu weist das Anschlussteil 4 an seinem proximalen Ende umfangsseitig ein Außengewinde 46 auf, auf dem die Überwurfmutter 44 aufgeschraubt wird. Um das Kupplungsteil 16 dem Anschlussteil 4 nur so weit zuzustellen, dass das Kupplungsteil 16 an dem Anschlussteil 4 flüssigkeitsdicht anliegt, das Kupplungsteil 16 dabei aber drehbar gegenüber dem Anschlussteil 4 und dem daran angeordneten Schaft 2 verbleibt, ist an dem Außenumfang des Anschlussteils 4 distalseitig des Außengewindes 46 ein ringförmiger Absatz 48 vorgesehen. Der Absatz 48 bildet einen Anschlag, gegen den die Überwurfmutter an dem Außengewinde 46 verschraubt werden kann, so dass das Kupplungsteil frei drehbar ist.

Ist die Überwurfmutter 44 auf dem Außengewinde 46 aufgeschraubt, kontaktiert eine Dichtlippe, die über den gesamten Umfang der dem Anschlussteil 4 zugewandten Stirnfläche des Dichtrings 38 angeordnet ist, eine proximalseitige Stirnfläche 50 des Drehschiebers 12. Auf diese Weise wird das Kupplungsteil 16 gegenüber dem Anschlussteil 4 abgedichtet.

An dem Kupplungsteil 16 ist proximalseitig eine Kupplung 52 angeordnet, die zum Befestigen des Arbeitseinsatzes 18 in dem Kupplungsteil 16 dient. Die Kupplung 52 ist wie bekannte Kupplungen dieser Art ausgebildet und daher hier nicht im Einzelnen beschrieben. Mittels der Kupplung wird der Arbeitseinsatz beim Einführen selbsttätig verriegelt, die Verriegelung kann entgegen Federkraft aufgehoben werden, wenn der Arbeitseinsatz entfernt werden soll.

### Bezugszeichenliste

- 2: Hohlschaft
- 4: Anschlussteil
- 6: Anschluss
- 8: Anschluss
- 10: Schieberventil
- 12: Drehschieber
- 14: Betätigungselement
- 16: Kupplungsteil
- 18: Arbeitseinsatz
- 24: Schlosskörper
- 26: Handhabungselement
- 28: Handhabungselement
- 30: Durchbrechung
- 31: Anschlusskontakt
- 32: Öffnung
- 33: Ansatz
- 34: Öffnung
- 36: Absatz
- 38: Dichtring
- 40: Absatz
- 42: Zweilochmutter
- 44: Überwurfmutter
- 46: Außengewinde
- 48: Ansatz
- 50: Stirnfläche
- 52: Kupplung

## Patentansprüche

1. Endoskopisches Instrument mit einem Hohlschaft (2), der proximalseitig mit einem Anschlussteil (4) mit zumindest einem Saug- und/oder Spülanschluss (6, 8) versehen ist, mit einem Kupplungsteil (16) zum Festlegen eines Arbeitseinsatzes (18), wobei das Kupplungsteil (16) gegenüber dem Anschlussteil (4) drehbar gelagert ist, und mit Mitteln (46) zur lösbaren Verbindung von Kupplungsteil (16) und Anschlussteil (4) sowie einer ringförmigen Axialdichtung (38), welche zwischen Kupplungsteil (16) und Anschlussteil (4) vorgesehen ist, wobei das Kupplungsteil Mittel (44) zur lösbaren Verbindung an dem Anschlussteil (4) des Instrumentes aufweist und drehbar gegenüber den Verbindungsmitteln (44, 46) zum Anschlussteil (4) ausgebildet ist, **dadurch gekennzeichnet, dass** das Kupplungsteil (16) distalseitig eine Axialdichtung (38) aufweist, welche mit dem Kupplungsteil (16) mitdreht und zur dichtenden Anlage an einer Stirnfläche (50) des Anschlussteils (4) ausgebildet ist.

2. Endoskopisches Instrument nach Anspruch 1, **dadurch gekennzeichnet, dass** die Axialdichtung (38) distalseitig am Kupplungsteil (16) angeordnet ist, mit dem Kupplungsteil (16) mitdreht und an einer Stirnfläche (50) des Anschlussteils (4) dichtend anliegt.

3. Endoskopisches Instrument nach einem der vorangehenden Ansprüche, **dadurch gekennzeichnet, dass** das Kupplungsteil (16) distalseitig einen hohlzylindrischen Abschnitt (33) aufweist, der abgestuft ausgebildet ist und der die Axialdichtung (38) trägt.

4. Endoskopisches Instrument nach einem der vorangehenden Ansprüche, **dadurch gekennzeichnet, dass** der Anschlussteil (4) ein Schieberventil (10) aufweist, welches den Hohlschaft (2) wahlweise mit einem Spül- oder Saugkanal (6, 8) verbindet, und dass die Axialdichtung (38) an einer Stirnseite (50) eines Drehschiebers (12) des Schieberventils (10) dichtend anliegt.

5. Endoskopisches Instrument nach einem der vorangehenden Ansprüche, **dadurch gekennzeichnet, dass** die Axialdichtung (38) durch eine Stirnfläche (36) eines abgestuften Teils des hohlzylindrischen Abschnitts (33) abgestützt ist.

6. Endoskopisches Instrument nach einem der vorangehenden Ansprüche, **dadurch gekennzeichnet, dass** die Mittel (44, 46) zur lösbaren Verbindung von Kupplungsteil (16) und Anschlussteil (4) eine am Kupplungsteil (16) angeordnete Überwurfmutter (44) und ein am Anschlussteil (4) angeordnetes Außengewinde (46) aufweisen, die gegen einen Anschlag (48) derart verschraubbar sind, dass das Kupplungsteil (16) gegenüber dem Anschlussteil (4) stets frei drehbar ist.

7. Endoskopisches Instrument nach einem der vorangehenden Ansprüche, **dadurch gekennzeichnet, dass** die Überwurfmutter (44) zwischen einer umfangsseitigen Stirnfläche des Kupplungsteils (16) und einem an einer Umfangsfläche des hohlzylindrischen Ansatzes (33) des Kupplungsteils (16) angeordneten Fixierring (42) formschlüssig gehalten ist.

8. Endoskopisches Instrument nach Anspruch 7, **dadurch gekennzeichnet, dass** der Fixierring (42) ein Innengewinde aufweist und an einem an einer Umfangsfläche des hohlzylindrischen Ansatzes (33) des Kupplungsteils (16) angeordneten Außengewinde angeschraubt ist.

9. Endoskopisches Instrument nach einem der vorangehenden Ansprüche, **dadurch gekennzeichnet, dass** der Hohlschaft (2) und eine dem Hohlschaft (2) zugewandte distalseitige Öffnung (32) des Kupplungsteils (16) fluchten und eine zweite Öffnung (34) des Kupplungsteils (16), welche proximalseitig angeordnet ist, exzentrisch zu der distalseitigen Öffnung (32) angeordnet ist.

## Claims

1. An endoscopic instrument with a hollow shank (2) which at the proximal side is provided with a connection part (4) with at least one suction connection and/or rinsing connection (6, 8), with a coupling part (16) for fixing a working insert (18), wherein the coupling part (16) is rotatably mounted with respect to the connection part (4), and with means (46) for the detachable connection of the coupling part (16) and connection part (4), as well as with an annular axial seal (38) which is provided between the coupling part (16) and the connection part (4), wherein the coupling part comprises means (44) for the detachable connection on the connection part (4) of the instrument and is designed in a rotatable manner with respect to the connection means (44, 46) to the connection part (4), **characterised in that** the coupling part (16) on the distal side comprises an axial seal (38) which co-rotates with the coupling part (16) and is formed for the sealing bearing contact on an end-face (50) of the connection part (4).

2. An endoscopic instrument according to claim 1, **characterised in that** the axial seal (38) is arranged on the coupling part (16) at the distal side, co-rotates with the coupling part (16) and sealingly bears on an end-face (50) of the connection part (4).

3. An endoscopic instrument according to one of the preceding claims, **characterised in that** the coupling part (16) at the distal side comprises a hollow-cylindrical section (33) which is designed in a stepped manner and which carries the axial seal (38).

4. An endoscopic instrument according to one of the preceding claims, **characterised in that** the connection part (4) comprises a slide valve (10) which selectively connects the hollow shank (2) to a rinsing channel or suction channel (6, 8), and that the axial seal (38) sealingly bears on an end-side (50) of a rotary slide (12) of the slide valve (10).

5. An endoscopic instrument according to one of the preceding claims, **characterised in that** the axial seal (38) is supported by an end-face (36) of a stepped part of the hollow-cylindrical section (33).

6. An endoscopic instrument according to one of the preceding claims, **characterised in that** the means (44, 46) for the detachable connection of the coupling part (16) and the connection part (4) comprise a union nut (44) arranged on the coupling part (16), and an outer thread (46) arranged on the connection part (4), and these can screwed against an abutment (48) in a manner such that the coupling part (16) is always freely rotatable with respect to the connection part (4).

7. An endoscopic instrument according to one of the preceding claims, **characterised in that** the union nut (44) is held with a positive fit between a peripheral-side end-face of the coupling part (16) and a fixation ring (42) arranged on a peripheral surface of the hollow-cylindrical lug (33) of the coupling part (16).

8. An endoscopic instrument according to claim 7, **characterised in that** the fixation ring (42) comprises an inner thread and is screwed on an outer thread which is arranged on a peripheral surface of the hollow-cylindrical lug (33) of the coupling part (16).

9. An endoscopic instrument according to one of the preceding claims, **characterised in that** the hollow shank (2) and a distal-side opening (32) of the coupling part (16) which faces the hollow shank (2) are aligned, and a second opening (34) of the coupling part (16) which is arranged at the proximal side, is arranged eccentrically to the distal-side opening (32).

## Revendications

1. Instrument endoscopique comprenant un arbre creux (2) pourvu, côté proximal, d'une pièce de raccordement (4) comportant au moins un raccord d'aspiration et/ou de rinçage (6, 8), un élément d'accouplement (16) pour la fixation d'un insert de travail (18), l'élément d'accouplement (16) étant monté à rotation par rapport à la pièce de raccordement (4), et des moyens (46) pour la liaison amovible de l'élément d'accouplement (16) et de la pièce de raccordement (4) ainsi qu'un joint d'étanchéité axial annulaire (38) prévu entre l'élément d'accouplement (16) et la pièce de raccordement (4), l'élément d'accouplement présentant des moyens (44) de liaison amovible avec la pièce de raccordement (4) de l'instrument et étant conçu mobile en rotation par rapport aux moyens de liaison (44, 46) avec la pièce de raccordement (4), **caractérisé en ce que** l'élément d'accouplement (16) présente, côté distal, un joint d'étanchéité axial (38) qui tourne avec l'élément d'accouplement (16) et est conçu pour porter de façon étanche sur une face avant (50) de la pièce de raccordement (4).

2. Instrument endoscopique selon la revendication 1, **caractérisé en ce que** le joint d'étanchéité axial (38) est disposé, côté distal, sur l'élément d'accouplement (16), tourne avec l'élément d'accouplement (16) et porte de manière étanche sur une face avant (50) de la pièce de raccordement (4).

3. Instrument endoscopique selon l'une des revendications précédentes, **caractérisé en ce que** l'élément d'accouplement (16) présente, côté distal, une partie cylindrique creuse (33) qui est conçue en gradins et porte le joint d'étanchéité axial (38).

4. Instrument endoscopique selon l'une des revendications précédentes, **caractérisé en ce que** la pièce de raccordement (4) présente une valve à tiroir (10) qui relie l'arbre creux (2) à, au choix, un canal de rinçage ou d'aspiration (6, 8), et **en ce que** le joint d'étanchéité axial (38) porte de manière étanche sur une face avant (50) d'un tiroir rotatif (12) de la valve à tiroir (10).

5. Instrument endoscopique selon l'une des revendications précédentes, **caractérisé en ce que** le joint d'étanchéité axial (38) s'appuie sur une surface avant (36) d'une partie en gradins de la partie cylindrique creuse (33).

6. Instrument endoscopique selon l'une des revendications précédentes, **caractérisé en ce que** les moyens (44, 46) pour la liaison amovible de l'élément d'accouplement (16) et de la pièce de raccordement (4) présentent un écrou d'accouplement (44) disposé sur l'élément d'accouplement (16) et un filetage extérieur (46) disposé sur la pièce de raccordement (4), lesquels sont aptes à être vissés contre une butée (48) de façon telle que l'élément d'accouplement (16) est toujours libre en rotation par rapport à la pièce de raccordement (4).

7. Instrument endoscopique selon l'une des revendications précédentes, **caractérisé en ce que** l'écrou d'accouplement (44) est maintenu par liaison de forme entre une surface avant périphérique de l'élément d'accouplement (16) et un anneau de fixation (42) disposé sur une surface périphérique de l'épaulement cylindrique creux (33) de l'élément d'accouplement (16).

8. Instrument endoscopique selon la revendication 7, **caractérisé en ce que** l'anneau de fixation (42) présente un filetage intérieur et est vissé sur un filetage extérieur disposé sur une surface périphérique de l'épaulement cylindrique creux (33) de l'élément d'accouplement (16).

9. Instrument endoscopique selon l'une des revendications précédentes, **caractérisé en ce que** l'arbre creux (2) et une ouverture (32) de l'élément d'accouplement (16), située côté distal et orientée vers l'arbre creux (2), sont en alignement et une deuxième ouverture (34) de l'élément d'accouplement (16), située côté proximal, est disposée excentriquement par rapport à l'ouverture (32) côté distal.
